# EUROPEAN PATENT APPLICATION

(11) **EP 2 354 236 A1**
(43) Date of publication of application: **10.08.2011**
(21) Application number: 10153121.8
(22) Date of filing: 09.02.2010
(51) Int. Cl.: C12P 3/00, B09B 3/00

(54) **Microbiological CO2 sequestration within waste disposal sites**

(71) Applicant: Biomim-Greenloop SA, 1000 Brussels (BE)
(72) Inventor: Wouters, Noémie, 9100, Sint Niklaas (BE); Valayer, Jean, 75012, Paris (FR); Zaoui, Caroline, 1030, Bruxelles (BE); Chapelle, Gauthier, 1090, Bruxelles (BE)
(74) Representative: Roufosse, Micheline C.

(57) **Abstract**

The present invention relates to the sequestration of large quantities of carbon dioxide generated in waste landfill sites. It provides means for sequestering and stabilising waste containing carbon and calcium by promoting the metabolism of calcifying microorganisms.

## Description

### FIELD OF THE INVENTION.

The present invention relates to the sequestration of large quantities of carbon dioxide generated in waste disposal sites. It provides means for sequestering and stabilising waste containing carbon and calcium by promoting the activity of calcifying microorganisms, typically bacteria and/or Archaea.

### BACKGROUND OF THE INVENTION.

The disposal of large amounts of waste requires large spaces. In addition it has a large environmental impact and presents several problems such as leaching or emission of greenhouse gases such as for example carbon dioxide, methane or nitrous oxide.

These problems have been addressed by various researchers and several methods have been offered to reduce or suppress them.

Typically, waste is disposed of in huge landfill disposal sites. These require a lot of space. In addition, because waste disposal is mostly unselective, it produces large amounts of toxic leachate that requires remediation

Methane emission can be either recovered, or removed by flaring. That method however is limited to methane reduction.

Another method, called dry tomb method, consists, in a first step, in stabilising and immobilising hazardous waste. In a second step, the waste volume is reduced. The stabilisation of waste though is not yet fully sustainable. In addition it requires chemical treatment and it is extremely costly. Another problem associated with that method resides in the absence of further bio-processing, thereby impeding extended waste valorisation.

Selective domestic waste disposal is presently compulsory in most large Western European cities. It is also required for most industrial waste disposal. It relies heavily on the good will of citizens and industries to comply with the required selection.

In yet another method, the degradation of organic material is accelerated by aeration with oxygen. The degradation of organic matter however releases carbon dioxide in the atmosphere, very little of which is presently sequestered.

It is also known to recirculate leachates in the landfill.

At a typical waste disposal site such as for example at Hong Kong NENT, WENT or SENT, the rate of waste deposition is of about 6,000 tons per day.

There is also a world-wide increasing demand for safe landfill sludge and dredged material disposal. For example, the port of Rotterdam dredges 20 Mm³ per year and the New York harbour has a deepening project requiring the dredging of 30 Mm³ of material, most of which needs to be disposed of. Incineration is typically used but there is a very high pressure for healthy landfills, leading to the possible reclamation of land area.

None of the methods described hereabove offers a satisfactory and efficient solution to the more and more acute problem of waste disposal and the problem will continue to grow as the planet gets more crowded.

There is thus a need to provide environmentally friendly and efficient methods to dispose of domestic and industrial waste.

### DESCRIPTION OF THE DRAWINGS.

Figure 1 represents typical decomposition pathways for the degradation of municipal waste.
Figure 2 is a diagram representing the fate of carbon derived from putrescible matter in domestic waste, and the possible production of carbonate. (Manning, 2001).

### SUMMARY OF THE INVENTION.

It is an objective of the present invention to dispose of carbon-containing waste which also contains calcium, or is supplemented with other calcium source or optionally alkaline waste, using calcifying and optionally sulphate and/or sulphate sulphur reducing microorganisms, typically bacteria or Archaea, in order to achieve carbon sequestration via biomineralisation.

It is also an objective of the present invention to promote and/or accelerate waste stabilisation

It is another objective of the present invention to sequester carbon-containing waste as precipitated carbonate.

It is a further objective of the present invention to provide a cost effective method for disposing of waste.

It is yet a further objective of the present invention to clean waste disposal sites and provide land suitable for reclamation.

Accordingly, the present invention fulfils any one of the foregoing objectives as defined in the independent claims. Preferred embodiments are defined in the dependent claims.

### DETAILED DESCRIPTION.

The present invention discloses a method for disposing of waste using calcifying microorganisms.

It discloses a method for sequestering carbon dioxide resulting from the degradation of waste by precipitation into carbonates using microorganisms having a calcifying activity, such as preferably heterotrophic microorganisms and optionally sulphate and/or sulphur reducing microorganisms.

More particularly, it discloses a method for disposing of waste that comprises the steps of:
a) selecting a landfill that comprises organic and/or biodegradable and/or inorganic waste material;
b) adding one or more heterotrophic calcifying microorganisms and optionally sulphate and/or sulphur reducing microorganisms;
c) optionally adding calcium-containing waste;
d) optionally adding alkaline waste
e) precipitating CO₂ released by degradation of organic or biodegradable matter as carbonate.

The landfill may comprise organic or biodegradable or inorganic material, or a combination thereof. The organic waste material can be selected for example from sludge, dredged material, oxalates, light organic acids or urea-rich waste, building material such as wood, domestic waste.

The metabolisms involved in the degradation of landfills have been described for example by Muthraparsad (Muthraparsad N., School of Molecular Cell Biology , 2007 or by Manning (Manning D.A.C., in Journal of Geological Society, 157, 229, 2000). If biologically maturing dredged materials and industrial sludge are mixed with a source of calcium ions, the metabolisms involved comprise:
- degradation of organic matter under aerobiosis;
- acidogenesis consisting in glucose fermentation leading to the synthesis of long chain fatty acids and a drop of pH and wherein the oxidation of said compounds leads to oxygen depletion and production of CO₂ and H₂;
- acetogenesis consisting in the degradation of organic acids to acetic acid leading to a further drop in pH and an increased production of H₂ which inhibits acetogens activity and promotes methanogens activity;
- methanogenesis consisting in the production of CH₄ either from acetate or from CO₂ and H₂ combined. Consumption of H₂ leads to an alkaline pH, thereby providing the possibility to precipitate carbon ions in the presence of calcium ions.

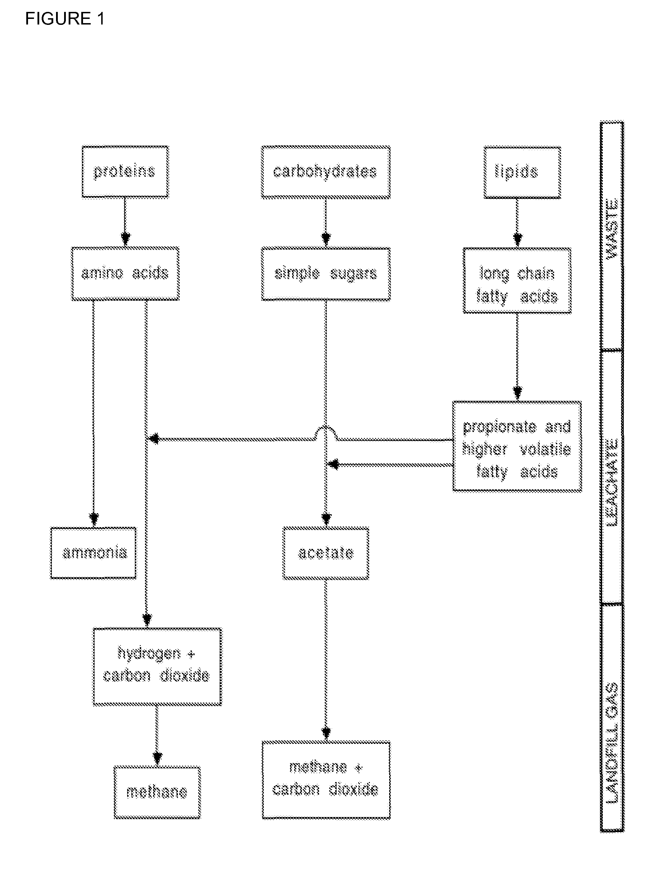

Generalised decomposition pathways for the dominant components of municipal waste, are represented in Figure 1. It emphasises the role of leachate as an intermediate stage in the production of landfill gas and as a sink for ammonia as ammonium as described for example by the Department of the Environment (1989) and .by Manning, D. A. C. (2000). "Carbonates and oxalates in sediments and landfill: monitors of death and decay in natural and artificial systems." Journal of the Geological Society 157, 229-238.

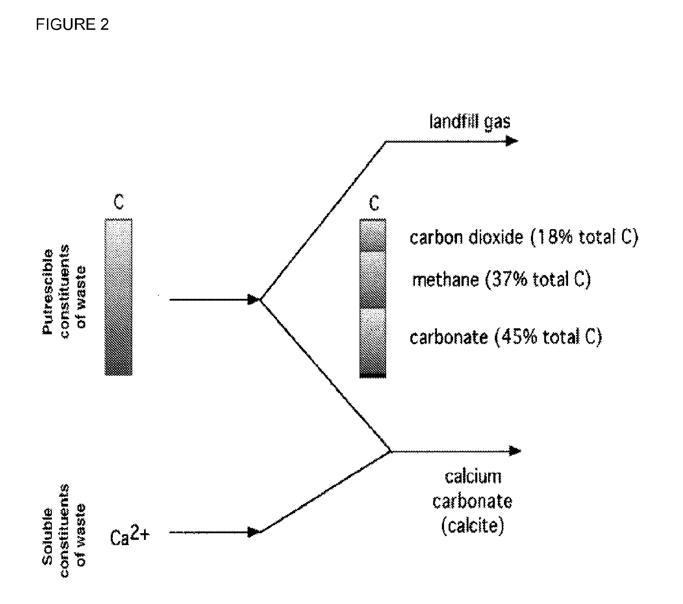

Figure 2 represents a conceptual diagram showing the fate of carbon derived from putrescible matter within domestic waste, maximising potential production of carbonate. As disclosed by Manning et al. (Manning, D. A. C. (2001). "Calcite precipitation in landfills: an essential product of waste stabilization." Mineralogical Magazine 65(5): 603-610.

Traditional anaerobic and aerobic techniques are compared in Rich et al. (Rich C., Gronow J, Voulvoulis N., in Waste Management, 28, 1039, 2008). The aerobic conditions are obtained by forcing air into waste mass. The anaerobic process comprises five stages: aerobic, fermentation, acetogenesis, methanogenesis and oxidation. During the process the temperature ranges between 30 and 65°C, preferably between 30 and 45°C in anaerobic conditions whereas it ranges between 40 and 70°C, ideally between 54 and 66°C in aerobic conditions. The pH ranges between 5 and 9, ideally 6.8 and 7.5 in anaerobic conditions whereas it ranges between 7.5 and 8.5 in aerobic conditions; In aerobic conditions, fewer acids are produced than in anaerobic conditions, resulting from limited fermentation. The time scale involved in the process vary from decades to millennia in the anaerobic process whereas it is reduced to at most 5 years in aerobic processes. The emissions are CO₂, H₂O and trace pollutants for both techniques with additional methane for anaerobic processes.

Different microbial guilds are capable of inducing and promoting biomineralisation, such as the generation and/or precipitation of carbonates, preferably via heterotrophic metabolisms and optionally via sulphate and/or sulphur reduction metabolisms. Hence, one or more calcifying microorganisms, that can be used in the present invention are preferably selected from but not restricted to, the families *Bacillaceae, Pseudomonadaceae, Enterobacteriaceae, Vibrionaceae,* and *Myxococcales* and optionally from one or more sulphate and/or sulphur reducing microorganisms such as, but not restricted to, the families *Desulfovibrionaceae* and *Desulfobacteriaceae.*

Said microorganisms may be indigenous to the waste material. Said microorganisms may also be isolated and selectively enriched outside the waste material. The microorganisms may be isolated and selected from environments where carbonate precipitation as well as sulphate and/or sulphur reduction have been shown to be the result of a microbial activity. The microorganisms may also be selected from strain libraries.

Calcium is needed in the process according to the present invention. If it is not present in the available waste material, it must be added, for example, as calcium-containing waste.

The calcium-containing supplement, selected for example from calcium-containing waste, is added in an amount ranging between 10 and 40 wt%, based on the total weight of waste. The amount added depends upon the nature of the waste to treat. It can be selected from gypsum and more preferably, under the form of sand or fine grains.

The alkaline waste, if present, can for example be selected from slag, fly ash or red mud, lime or gypsum. It can be added either as bottom liner or added periodically. It is preferably added in an amount sufficient to reach a pH of at least 7, preferably of from 7 to 10 and more preferably of 8 to 9. The pH is measured on the leachate. The microorganisms must be placed in conditions allowing the promotion of preferably heterotrophic metabolisms and optionally sulphate and/or sulphur reduction, that lead to carbonate formation and precipitation.

The microorganisms, such as bacteria and Archaea, selected according to the present invention, promote and accelerate waste stabilisation by reaction with the carbon dioxide resulting from degradation of organic or biodegradable matter. They promote the precipitation of carbon dioxide into carbonate, preferably calcium carbonate.

The precipitation reaction is preferably carried under moist conditions. The preferred level of humidity is of at least 60%.

It is also preferred that the precipitation is carried under aerobic conditions.

The present method thus results in the sequestration of large quantities of carbon waste through their stabilisation as solid carbonate. More preferably, carbon dioxide is trapped and sequestered as calcium carbonate.

Hazardous waste is thereby stabilised into a solid matrix formed of calcite or other forms of calcium carbonate. It offers the further advantage of immobilising heavy metals possibly contained in waste.

Landfill greenhouse gas emissions are also reduced by sequestration of carbon dioxide.

In a preferred embodiment according to the present invention, the leachates are recycled.

Because the method used in the present invention is clean and ecologically friendly, land reclamation is an option possible on a clean disposal site soil.

The mechanical strength of micro-organism-induced carbonate precipitation is excellent when mixed with sand as explained by Al-Thawadi (Al-Thawadi, S, in Biological Sciences and Biotechnology, Murdoch University, Perth, AU, 2008). The deposits, preferably mixed with sand, can thus provide sound foundations for construction. In addition, mechanical strength of bio-remediated sludge and dredged material can increase during the ripening phase, as disclosed by de Haan et al. (de Haan et al., in Water Science and technology, 37, 371, 1998). Said ripening phase includes physical processes such as desiccation and chemical and biological processes. This structure can be reinforced by addition of cement or additives causing mineral precipitation such as carbonates as disclosed in Wu et al. (Wu T.H., Zhou S.Z., Gale S.M., in Canadian geotechnical Journal, 44, 545, 2007). Alternatively, it can be strengthened by pH manipulation or microbial acceleration as explained by Burnham in US-A-5,417,861

Landfill wastes are a significant source of oxalates and light acids. They can be degraded with precipitation of calcite in the presence of a source of calcium ions as described by Manning (Manning D.A.C., in Journal of Geological Society, 157, 229, 2000). It is known that the evolution of leachate is methanogenic and releases carbon dioxide. In the presence of ammonium ions resulting from denitrification, pH increases thereby causing precipitation of carbonates. Precipitation of calcite as cement thus appears also in landfill processes of stabilisation.

Maximising carbon sequestration efficiency of biologically maturing dredged material and sludge can be obtained by the present invention. By providing buffered conditions, or additional alkalinisation, or soil amendment with chicken or green manure, or addition of gypsum or lime, optimal conditions can be created.

In summary the present invention provides an accelerated land curing system as well as sustainable waste management. It is cost efficient and does not require any gas collection system.

### EXAMPLES.

### Example 1.

Treatment of landfill containing 60 wt%, based on the total weight of waste, of organic and biodegradable material comprising:
- addition of calcifying microorganisms;
- Addition of 10 to 40 wt%, based on the total weight of waste, of calcium containing waste selected from gypsum, under the form of fine grains;
- Addition of alkaline waste selected from slag, fly ash or red mud or lime in order to reach a pH of at least 7;
- In aerobic landfill and with a degree of humidity of at least 60%

At typical waste disposal sites such as for example at Hong Kond NENT, WENT or SENT landfills, the rate of deposition is of about 6,000 t/day. After a period of time of 5 years, which is the average time of completion for an aerated landfill, 11 Mt of waste have been deposited. A maximum amount of 7.5 Mt of CaCO₃ is thus produced. It corresponds to about 70 % of waste stabilisation by formation of calcite. This corresponds to the sequestration of 3 Mt of carbon dioxide over a period of time of 5 years.

### Example 2.

Treatment of predominantly inorganic landfill containing at most 10 wt%, based on the total weight of waste, of organic and biodegradable material comprising:
- addition of calcifying microorganisms
- Addition of 10 to 40 wt%, based on the total weight of waste, of calcium containing waste selected from gypsum, or calcium containing waste under the form of fine grains;
- Addition of alkaline waste selected from slag, fly ash or red mud or lime in order to reach a pH of at least 7
- In aerobic landfill and with a degree of humidity of at least 60%

At typical waste disposal sites such as for example at Hong Kond NENT, WENT or SENT landfills, the rate of deposition is of about 6,000 t/day. After a period of timeof 5 years, which is the average time of completion for an aerated landfill, 11 Mt of waste have been deposited. A maximum amount of 1.2 Mt of CaCO₃ is thus produced. It corresponds to about 11 % of waste stabilisation by formation of calcite. This corresponds to the sequestration of 0.5 Mt of carbon dioxide over a period of time of 5 years.

## Claims

1. A method for sequestering carbon dioxide resulting from the degradation of waste by precipitation into carbonates using calcifying microorganisms.

2. The method of claim1 that comprises the steps of:
a) selecting a landfill that comprises organic and/or biodegradable and/or inorganic waste material;
b) adding one or more calcifying microorganisms with preferably heterotrophic metabolisms and optionally sulphate and/or sulphur reducing metabolisms;
c) optionally adding calcium-containing waste;
d) optionally adding alkaline waste
e) precipitating CO₂ released by degradation of organic matter as carbonate.

3. The method of claim 1 or claim 2 wherein the calcifying micro-organisms are selected from either natural or artificial environments.

4. The method of any one of the preceding claims wherein calcium-containing waste is added in an amount of 10 to 40 wt%, based on the total weight of waste.

5. The method of any one of the preceding claims wherein the calcium-containing waste is selected from gypsum or anhydrate and is preferably added under the form of fine grains.

6. The method of any one of the preceding claims wherein alkaline waste is added in an amount sufficient to obtain a leachate having a pH of at least 7, preferably from 7 to 10.

7. The method of any one of the preceding claims wherein alkaline waste is selected from slag, fly ash, lime or red mud.

8. The method of any one of the preceding claims wherein the disposal process is carried out in aerobic landfills.

9. The method of any one of the preceding claims wherein the disposal process is carried out under a level of humidity of at least 60%.

10. The method of any one of the preceding claims further comprising the recycling of leachates.

11. Use of microorganisms having a calcifying activity to react with the carbon dioxide resulting from degradation of organic or biodegradable matter and to precipitate carbon dioxide into carbonate.

12. Use of the waste landfill sites treated by the method of any one of claims 1 to 9 for land reclamation.
